# EUROPEAN PATENT APPLICATION

(11) **EP 4 582 439 A1**
(43) Date of publication of application: **09.07.2025**
(21) Application number: 22956700.3
(22) Date of filing: 01.09.2022
(51) Int. Cl.: C07K 14/46, C07K 14/78, C08H 1/00, C08J 3/24, A61K 9/00

(54) **METHOD FOR OBTAININGMETHOD FOR OBTAINING A POROUS INJECTABLE SCAFFOLD BASED ON SIMILAR BIOPOLYMERS WITH DIFFERENT MELTING TEMPERATURES**

(71) Applicant: Cells For Cells S.A., Santiago 7550101 (CL)
(72) Inventor: ZAVALA VIVAR, Gabriela, Santiago, 7550101 (CL); HIDALGO MANOSALVAS, Carmen, Santiago, 7550101 (CL); KHOURY, Maroun, Santiago, 7550101 (CL); ACEVEDO COX, Juan Pablo, Santiago, 7550101 (CL)
(74) Representative: Elzaburu S.L.P.
(86) International application number: PCT/CL2022/050086
(87) International publication number: WO 2024/044863

(57) **Abstract**

Method for generating a porous injectable scaffold that includes providing a liquid composition of 2 phases at a temperature below 25°C, of Newtonian behavior, where the composition comprises:
a liquid dispersant phase at room temperature formed by a gelatin with a low melting point, less than 15°C, functionalized with methacryloyl or methacrylamide groups; and a photoinitiator;
and a dispersed phase, of microdroplets or beads in solid state, of a gelatin solution with a melting point greater than 25°C;
initiating the polymerization of the dispersing phase by light radiation; raising the temperature to 35-40°C and allowing melting of the dispersed phase; and obtaining a porous scaffold.

The formed porous scaffold and its use as a biological support for tissue regeneration/generation; as a biological matrix as a support for cells, for cell invasion; as an acellular biological matrix, a biological matrix as a mechanical support and/or a biological matrix for active components.

## Description

### Technical field

The present invention relates to the biomedical field, in particular it refers to a method for obtaining an injectable porous scaffold, as a biological matrix, based on similar biopolymers, but with different melting temperatures and the use of said injectable scaffold.

### Background

Tissue and organ replacement is considered the only feasible treatment for many critical illnesses. In the context of the shortage of organ and tissue donors, the development of new tissues or functional organs is a priority issue for public health. Tissue engineering has emerged as an ideal technology for this endeavor. Tissue engineering is a promising area that aims to reduce the imbalance in organ and tissue supply.

Tissue engineering must consider many critical factors, including scaffolds and biological mediators, to mimic the cells' native environment and replace damaged tissue. In this context, scaffold design must consider the physical and biological aspects of tissues for proper signaling towards cell maintenance, differentiation, and tissue regeneration. In the case of physical aspects, porosity and stiffness can be modified. In terms of biological factors, biocompatibility, cell infiltration, cell adhesion, encapsulation or binding of growth factors, and incorporation of auxiliary cells can be explored to improve the performance of biomaterials to repair, replace, or induce the regeneration of injured tissues and organs.

Scaffolds are three-dimensional arrangements of biomaterials capable of mimicking the native extracellular matrix (ECM) and facilitating cell expansion and/or the pattern or arrangement of different cell types, matrix deposition, and tissue production. In this sense, one of the most promising biomaterials are hydrogels. They are composed of a hydrophilic polymer network capable of absorbing water molecules through hydrogen bonds, polar and ionic interactions. Hydrogels can mimic ECM and be used in biomedical applications such as drug delivery and tissue engineering. Depending on the application, hydrogels have different forms of administration, such as implantation, dressing, oral dose, etc. However, implanting preformed hydrogels requires an invasive procedure that can cause pain, a long recovery time after the operation, and a high cost. Therefore, researchers have focused on developing injectable hydrogels to overcome the drawbacks of administering preformed hydrogels.

Injectable hydrogels would be an ideal system for *in vivo* implantation at the target site through minimally invasive procedures that can be adapted to the full extent and shape at the site of injury. The term injectable means that the hydrogel is formed *in situ* by a solution-gel (sol-gel) transition after using an extrusion device to implant the solution (sol) or pre-gel at the target site.

There are several requirements in the design of an injectable hydrogel for tissue engineering. In addition to adequate injectability, the scaffold must be biodegradable, allowing for a gradual replacement of the biomaterial with a tissue-like ECM, material that is secreted by cells (remodeling) while keeping the cells at the site of injury. The scaffold must be biocompatible and minimize severe and chronic inflammatory responses. In addition, the scaffold requires mechanical properties to withstand physiological loads and must have adequate porosity to facilitate cell migration and the exchange of nutrients and waste.

During the formulation of injectable hydrogels, combination with a therapeutic agent could enhance the regeneration repair of the target tissues. However, injectable hydrogels must meet the following requirements: low viscosity to prevent clogging of the syringe nozzle during extrusion; The crosslinking reaction should be fast enough to achieve the solution-gel transition quickly, avoiding the outflow of materials into the surrounding tissue after injection, and finally, the sol-gel transition should not involve toxic or harmful processes.

Biocompatibility, defined as the ability of a material to simultaneously promote desirable functions for a specific application without eliciting a chronic and severe inflammatory response or damage to cells, is a key design factor for the engineering of injectable hydrogels. The main challenge for the *in vivo* biocompatibility of injectable hydrogels is the degradation of the hydrogels without harmful stimuli, toxic reagents or resulting in toxic byproducts. Depending on the tissue to be designed or the biomedical application, a desired hydrogel degradation kinetics should be considered in the scaffold design. The degradation of the material in a precise space and time should reflect the rate of new tissue formation or the controlled release of bioactive molecules.

### The effect of scaffolding stiffness

It is well known that tissues and organs vary in elasticity from 1 kPa in the brain to 20 GPa in cortical bone. It is therefore not surprising that mechanical and physical elements of the environment can influence cell behavior, such as cell propagation, migration, proliferation, and differentiation through mechanical stimuli. For example, soft scaffolds that mimic the mechanical properties of the brain are suitable for directing stem cell differentiation to the neurogenic lineage. In contrast, stiffer scaffolds that mimic the mechanical properties of muscle allow cells to move toward a myogenic lineage. Rigid scaffolds that mimic bone collagen are suitable for directing cells to the osteogenic lineage.

Designing an injectable hydrogel as a robust mechanical system is a huge challenge. Not only because few hydrogels present rigidity in the range of the body's structural tissues, but also because the appropriate parameters for injectability are counteracted if the scaffold or hydrogel has been designed to resist mechanical stresses once implanted.

### PREVIOUS STATE OF THE ART

In the state of the art there are different solutions to the technical problem addressed by the invention.

The inventors, in a previous patent WO2017216780A1, have developed an injectable scaffold based on gelatin with a low melting point that solves many of the technical problems of an injectable scaffold: biocompatibility, easy handling by the surgeon since at room temperature the solution has a Newtonian behavior, and the appropriate rigidity of the scaffold once polymerized. However, the inventors noticed that tissue regeneration with this first solution was not as good or fast, presumably because of the difficulty of the cells to integrate into the compact rigid structure. To solve this technical problem, the inventors developed the protected solution in this application, where a porous scaffold is generated. While the main focus is the generation of the porous scaffold *in situ* in the biomedical field, this new development can also be implemented in other applications, such as the 3D printing of porous scaffolds.

Other publications have explored the formation of porous scaffolds in a mold, for example, Zoratto, Nicole, et al (Bioengineering & Translational Medicine 5.3 (2020): e10180), disperse porcine methacrylicated gelatin (GelMA) in oil, cross-linked at 4°C, and exposed to UV light in a mold. In this case of application in biomedicine, the porous scaffold should be implanted only once it has been formed, and therefore it would not be completely adapted to the injury. Additionally, within the previous art we find the technology requested in the US20140079752A1 patent. This technology uses a solid porogenic element that differs from the injectable liquid phase in that the latter degrades at least 10% slower than the former after implantation and polymerization. In this technology, it requires first the digestion of the porogen before a pore is observed.

The invention stands apart from previous art and describes a new method for forming an injectable porous scaffold, useful for tissue repair and regeneration.

### Figures.

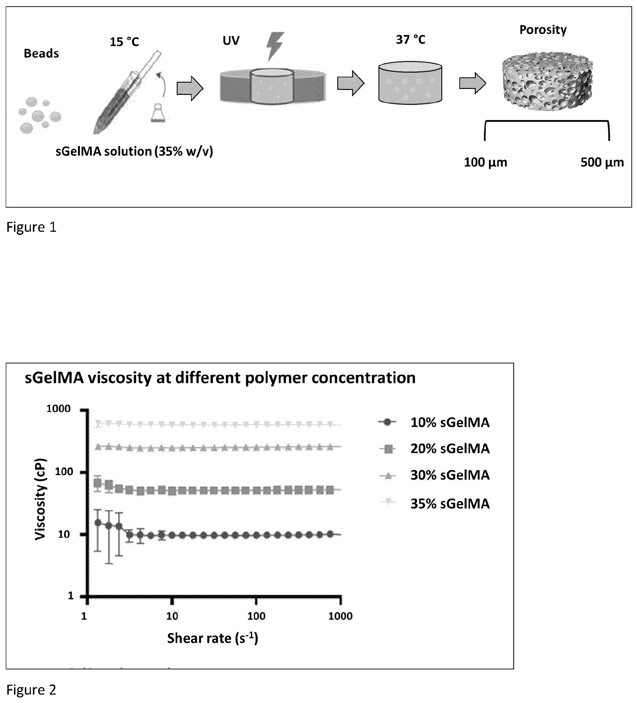

### Figure 1. Schematic representation of the method of invention.

**Figure 2****. SGelMA viscosity at different polymer concentrations.** Between 10 to 35% (w/v) of methacrylicated salmon gelatin (sGeIMA). (n=9 three independent experiments in triplicate).

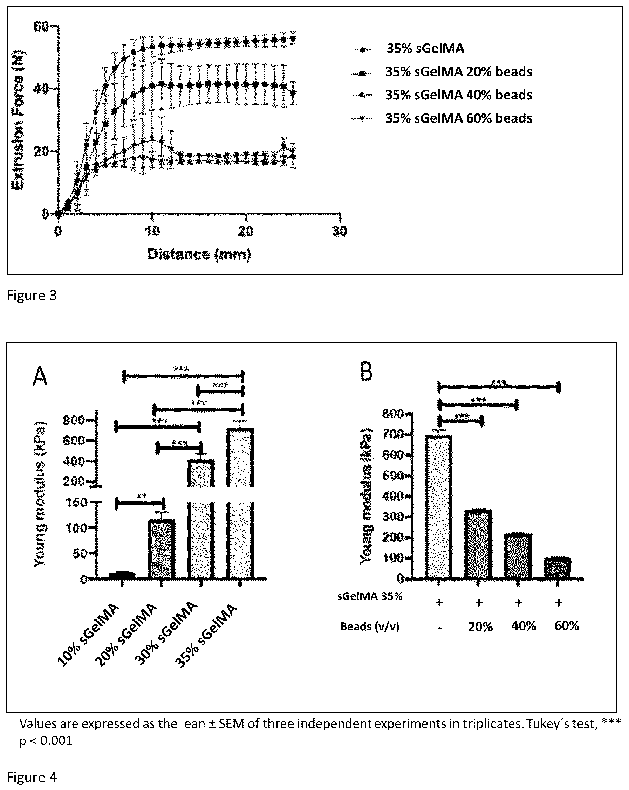

**Figure 3****. sGelMA injectability strength at 35% w/v of sGelMA with different bead concentrations: 20, 40 and 60% (v/v) at 25°C.** The values are expressed as the mean ± SE (Standard Error) of three independent experiments in triplicate
**Figure 4. 4-A** **Mechanical compressive strength after photopolymerization for SGelMA** at **different concentrations: 10%, 20%, 30% and 35% (w/v).** 4-B Mechanical compressive strength after photopolymerization of SGelMA at 35% with different concentrations of porcine gelatin beads: 0, 20%, 40% and 60% (v/v) (n=9 three independent experiments in triplicate).

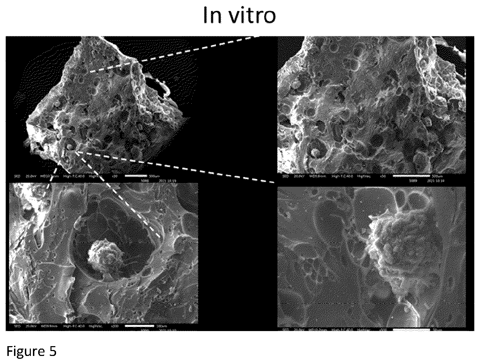

**Figure 5****. Scanning electron microscopy photographs** of the porous scaffold of the invention formed *in vitro* with an amplification of A) 30X, B) 50X, C) 200X and D) 500X.

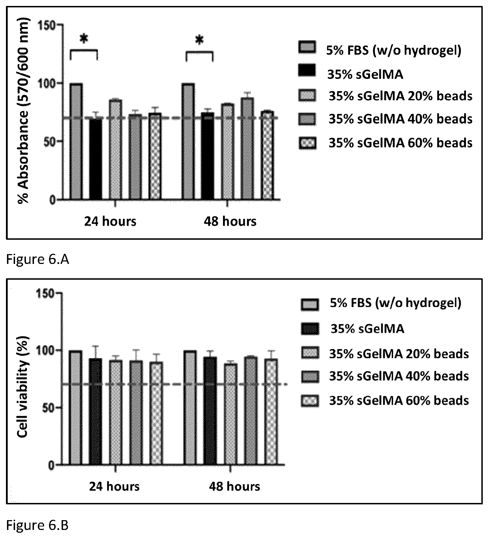

**Figure 6****. Effect of porous sGelMA extracts on metabolic activity and cell viability.** (A) Metabolic activity of 3T3 fibroblasts after 24 and 48 hours of exposure with sGelMA porous hydrogel extracts. (B) Viability of 3T3 fibroblasts after 24 and 48 hours of exposure with sGelMA porous hydrogel extracts. (a) and (b) are normalized to control with cell culture medium. The values are expressed as the mean ± SE of three independent experiments in triplicate. Kruskal-Wallis test, * p < 0.05; ** p < 0.01; *** p < 0.001.

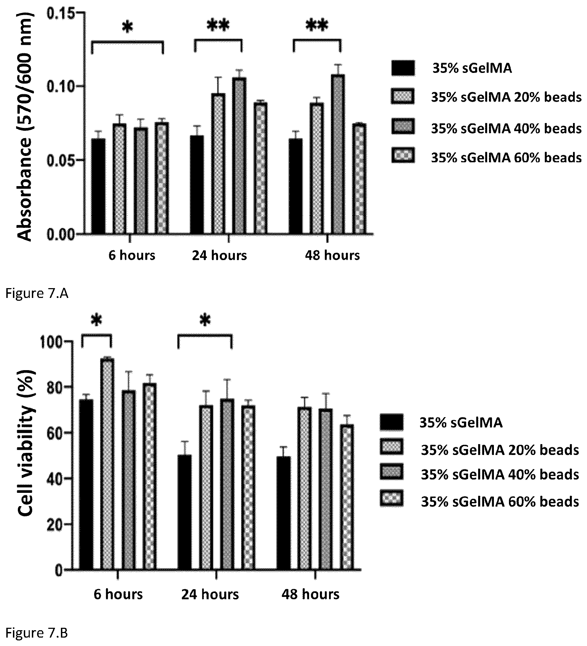

**Figure 7****. Viability of cells encapsulated in sGelMA porous hydrogels.** (A) Quantification of live cells against total number of cells and (B) the metabolic activity of cells after 6, 24 and 48 hours in sGelMA porous hydrogels. The values are expressed as the mean ± SE (at least n=4 per condition). Kruskal-Wallis test, * p < 0.05; ** p < 0.01; *** p < 0.001.

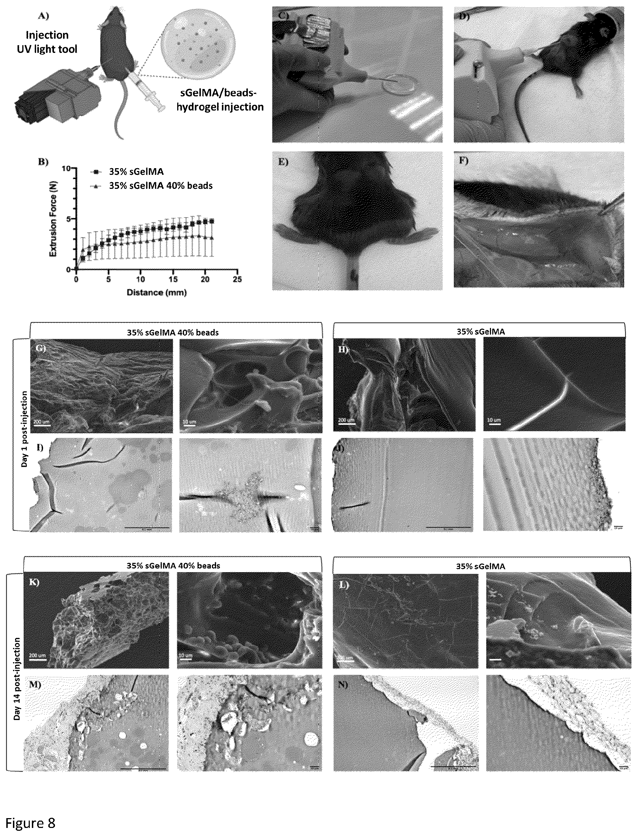

**Figure 8****. Injection of porous and non-porous sGelMA hydrogels.** (A) Schematic representation of subcutaneous injection of porous and non-porous SGelMA formulations and *in situ* crosslinking. The porous formulation includes 35% (w/v) salmon GelMA combined with 40% (v/v) porcine beads. The non-porous formulation includes 35% salmon GelMA (w/v). (C) The prototype UV light allows for in-situ polymerization via a retractable optical fiber integrated within an 18 G x 1" needle. (D). Photocrosslinking of the sGelMA formulation using the UV light injectable tool. (E) Visualization of two hydrogels injected subcutaneously into the back of a mouse. (F) Construct of hydrogel collected after injection and crosslinking. Representative electron microscopy images of porous (G) and nonporous sGelMA hydrogels after 1 day after implantation (H). Scale bar: 200 µm and 10 µm. Hematoxylin-eosin staining of porous (I) and non-popped (J) explants after 1 day of injection. Scale bar: 0.1 mm. Representative electron microscopic images of porous sGelMA (K) and nonporous hydrogels after 14 days after injection (L). Scale bar: 200 µm and 10 µm.Hematoxylin-eosin staining of porous (M) and non-pouring (N) explants after 14 days of injection. Scale bar: 0.1 mm.

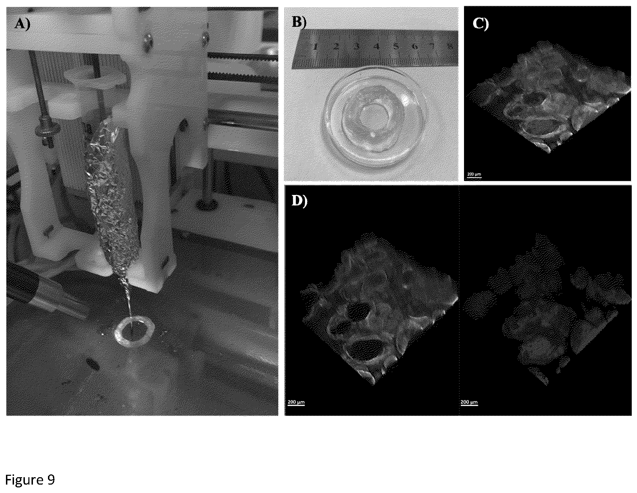

**Figure 9****. 3D printability using a salmon GelMA bioink with gelatin beads.** (A) 3D printing of a construction using the 3D printer model Fab@Home and GelMA of salmon at 35% (w/v) with porcine gelatin beads (40% v/v). (B) A doughnut-shaped 3D construction after printing and UV cross-linking. (C) Representative image of beads visualized in red fluorescence (rhodamine). Scale bar: 200 µm. (D) Representative image of a 3D construction, visualizing bulk hydrogel with green fluorescence (BSA-FITC) and beads with red fluorescence (rhodamine). Scale bar: 200 µm.

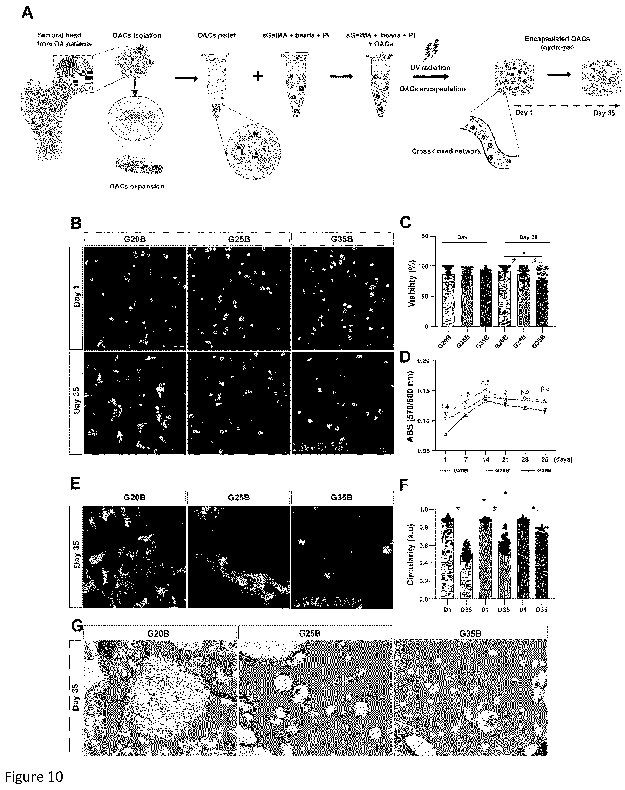

**Figure 10****. Viability and distribution of OACs encapsulated in G20B, G25B and G35B (the number represents the concentration of sGelMA in the pre-polymerized bioink or hydrogel, and the letter B represents that the bioink has a concentration of 40% v/v of porcine gelatin beads).** A) shows a large proportion of living cells and a change in morphology from a rounded phenotype to a fibroblastoid one. Scale bar = 100 µm.B) LIVE/DEAD^{®} staining on days 1 and 35 after encapsulation. C) Quantification of cell viability by counting live and dead cells (each point represents the mean value of a cross-section of the hydrogel n = 3 OAC donors). D) Metabolic activity, as another measure of viability, was assessed weekly by the Prestoblue assay and shows that the reduction of rezasurin had an increase after 2 weeks and then remained constant with the exception of G35B. n= 3 hydrogels per formulation, 4 OAC donors. The data are shown as a mean ± SEM and were analyzed with 2-way ANOVA and Tukey's post-test, *p < 0.05 (α: G20B vs G25B; β: G20B vs G35B; ϕ: G25B vs G35B). E) The alpha-SMA stain at 35 days shows the change in morphology that OACs undergo over time. Scale bar = 100 µm. F) Circularity of OACs in G20B, G25B and G35B after 1 and 35 days of encapsulation (each dot represents the mean of 1 photo, n = 3 donor OACs). G) H&E staining shows the homogeneous distribution of OAC in hydrogels after the differentiation protocol.

### Detailed description of the invention.

The invention refers to a method to generate a porous scaffold, adaptable to the shape required, where an injectable composition is provided, with Newtonian behavior, which adapts perfectly to any shape, for example, of a lesion to be repaired, and that once at the destination site solidifies forming a porous scaffold that acts as a support for cells, allowing tissue regeneration.

This method involves providing a 2-phase injectable composition, where the composition includes:
a first liquid dispersant phase at room temperature (6-25°C) formed by a low-melting point gelatin solution determined because it has a proline/hydroxyproline content of 18% or less with respect to the total amino acid content; which is additionally functionalized with methacryloyl or methacrylamide groups, and a polymerization photoinitiator;
and a second dispersed phase, solid at room temperature (6-25°C), of microdroplets or beads of a gelatin solution with a melting point close to 35°C, which has a proline/hydroxyproline content of 20% or more with respect to the total amino acid content.

Conveniently, the dispersing phase has a gelatin originated from fish adapted to cold environments, such as salmon or trout, and the dispersed phase corresponds to mammalian gelatin beads, such as porcine or bovine gelatin.

The polymerization photoinitiator is a free radical generator that is activated by light radiation, such as: 2-hydroxy 4'(2-hydroxyethoxy)-2-methylpropiophenone (Irgacure^{®} 2959), or lithium phenyl-2,4,6-trimethylbenzoyl phosphine (LAP), or any equivalent compound, which is preferably used in a concentration of approximately 0.01% to 5% (w/v).

The composition is handled at a temperature between 8 and 25°C, where the gelatin of the dispersant phase is liquid and presents a Newtonian behavior, that is, it behaves as a liquid with a stable viscosity independent of the forces exerted on it, completely flooding any shape of the container where it is poured. This characteristic is of vital importance and very advantageous in the field of tissue repair using porous scaffolds such as the one in the present invention, since until now these scaffolds are produced outside the tissue and are introduced already assembled, trying to adapt them to the target area. This is laborious and often impossible, given the inherently irregular nature of injuries. This is solved with the present invention, where in one realization the scaffold is formed within the same lesion, fitting perfectly with the entire lesion, allowing a complete coverage of it, thus facilitating complete regeneration in the treated organ or tissue.

In one embodiment, the described composition is injected by any available means, for example, a syringe, or conveniently by an apparatus comprising both an injection channel of the composition and a source of light radiation. If you do not have a device of these characteristics, a common clinical syringe and a different device that generates a light emission can be used. Since the composition has a Newtonian behavior, its application is simple and does not require large access channels to reach tissues or organs within the body and that otherwise could generate pain or discomfort after implantation.

Once at the destination, the polymerization of the dispersant phase with light radiation must begin, according to the photoinitiator used. For the expert in the technique, it will be evident that the polymerization generated with the functionalized gelatin and the photoinitiator is complete and irreversible, so a network of the entire dispersing phase interrupted by the dispersed phase is generated. The dispersed phase, having a melting point of between 30 and 35°C, will begin to fuse (melt) naturally due to the temperature of the target tissue, and to drain from the scaffold, thus leaving a porous scaffold formed *in situ,* of the exact shape of the lesion to be treated, without empty spaces, or irregularities that can generate discomfort.

To develop the method of the invention, the inventors started from the conceptual idea of generating a formulation that includes two solutions of gelatins with different melting points. This formulation allows an injectable porous scaffold to be generated to fill or treat damaged tissues through minimally invasive procedures, such as injection or arthroscopy.

One of the gelatin solutions (unmodified mammalian gelatin) in the form of gelled microbeads (solid state) is combined with a liquid solution of low-melting gelatin, e.g. salmon, methacrylic, (methacrylate salmon gelatin, sGeIMA). The solid-liquid configuration of this mixture is stable due to its large difference in melting point (32°C vs 4°C respectively). Therefore, at room temperature (15-25 °C), the microbeads remain in a solid state, while sGelMA or dispersant solution remains liquid.

Although experiments were carried out using salmon gelatin, for the expert in the art it will be obvious that the species of origin of the gelatin is not the important thing, but the condition that said gelatin is liquid at a temperature between 8 and 25°C, as the inventors have already established in previous works (WO2017216780 A1) this condition correlates with the percentage of proline and hydroxyproline of the gelatin, where the proline/hydroxyproline content is of 18% or less with respect to the total amino acid content. This proportion occurs naturally in fish such as salmon, but gelatin from another marine species that live in cold waters could be used or even a gelatin that meets these characteristics could be artificially produced. In the same way, the origin of the mammalian gelatin used is not relevant either, but the condition of maintaining a solid state at temperatures below 25°C and liquid at physiological temperature, that is, from 35° onwards, or suitably liquid above 30° C.

In one embodiment, this stable composition of the invention is implanted into a lesion by injection or arthroscopy (in the case of joint defects), and immediately irradiated with UV light to induce irreversible covalent crosslinking and solution-gel transition of the sGelMA dispersing phase. This new method would make it easier to fill the lesion or defect that needs to be restored with a hydrogel. Subsequently, and driven by physiological temperature, the high-melting gelatin microspheres will undergo a transition from solid to liquid leaving a porous scaffold within the lesion. A diagram of the invention can be seen in **Figure 1****.**

Thus, the invention refers to a method for generating a porous scaffold as a biological matrix, which includes the stages of:
i) providing a liquid composition of 2 phases at a temperature below 25°C, of Newtonian behavior, where the composition comprises:
   a first liquid dispersant phase at room temperature formed by a gelatin with a low melting point, less than 15°C, determined by the fact that it has a proline/hydroxyproline content of 18% or less with respect to the total amino acid content; which is additionally functionalized with methacryloyl or methacrylamide groups; and a photoinitiator;
   and a second dispersed, solid-state microdroplet or bead phase of a gelatin solution with a melting point greater than 25°C, determined by having a proline/hydroxyproline content of 20% or more of the total amino acid content;
(ii) extruding the solution provided at the site where the porous scaffold will be formed;
(iii) initiating the polymerization of the liquid dispersant phase by applying light radiation;
(iv) raising the temperature to 35-40°C and allow the melting of the microdroplets or beads of the dispersed phase; and
v) obtaining a porous scaffold by gelling the dispersing phase and discarding by melting and/or diffusion of the dispersed phase.

In addition, the liquid dispersant phase additionally comprises active compounds such as drugs, growth and/or migration factors, cells, organelles, antibodies, peptides, vesicles, cell derivatives, oligonucleotides or others.

Where the low-melting point gelatin of the dispersing phase is at a concentration between 10 and 50 % w/v; and the degree of functionalization with methacryloyl or methacrylamide groups of the gelatin polymer or gelatin with a low melting point of the dispersant phase is 30% to 100% in the lysine residues of the amino acid chain of said gelatin polymer.

On the other hand, gelatin beads of the dispersed phase have a concentration of between 5% and 30% w/v of gelatin; they have a diameter of between 50 and 500 µm; and they occur at a concentration between 10 and 80% (v/v) within the dispersing phase. In a preferred realization, the concentration of the beads in the dispersing phase is between 20 and 60% (v/v).

In one realization in stage ii) the composition is injected directly into the site where the formation of the recipient scaffold, tissue, or lesion is required, and in stage iii) it is polymerized in situ. That is, it is injected onto a lesion, and given the Newtonian character and low viscosity of the composition, it is easily injectable, adapts and completely covers the lesion, and when polymerized, a porous scaffold perfectly adapted to the required shape is formed; where the natural temperature of the recipient tissue allows the melting of the beads of the dispersed phase.

In another realization in stage ii) the composition is extruded by a 3D printing system, which incorporates a system for the application of UV light or light emission for the polymerization of stage iii. In this case, the application of heat to raise the temperature, according to stage iv) can be done *in vitro,* with an external source of heat. Or in a second modality of this realization, the application of heat to raise the temperature, stage iv) can be carried out naturally by the temperature of the tissue, by placing the scaffold, polymerized in a 3D printing system, on a lesion or receiving tissue.

Conveniently, low-melting point gelatin is derived from organisms of the genus Salmo or Oncorhynchus, and solid gelatin at room temperature is porcine or bovine gelatin.

In one embodiment, the invention refers to the porous scaffold formed according to the method described, where said scaffold is formed of polymerized gelatin with mesopores between 50 and 500 µm in diameter. Optionally, the scaffold comprises active compounds such as drugs, growth and/or migration factors, cells, organelles, antibodies, peptides, vesicles, cell derivatives and/or oligoucleotides

This scaffold is useful as a biological support for tissue regeneration/generation; where tissue regeneration is in a cartilage injury, bone injury, ulcerative injury, ligament injury, organ injury, spinal cord injury, or soft tissue injury, or in a reconstitution of skin tissue, muscle tissue, soft tissue, or post-surgical replacement tissue.

In another embodiment, the invention focuses on the use of the scaffold formed as a biological matrix as a support for cells, for cell invasion. And in another embodiment, the invention points to the use of the scaffold formed as an acellular biological matrix, biological matrix as a mechanical support, or biological matrix for active components.

Regarding the stage of raising the temperature, this can be done naturally *in situ,* with the patient's body temperature or, in other words, the tissue in which the composition is applied. The phase dispersed by its bovine or porcine gelatin characteristics may be degraded by the patient's body. The scope of the invention will be better understood in the light of the following illustrative examples of the invention.

### Examples

### Example 1. Functionalization of Salmon gelatin with methacryloyl and methacrylamides groups.

First, we obtained purified and filtered salmon gelatin that is then dried and ground.

The crushed salmon gelatin was dissolved to a desired final concentration of 10% to 35% (w/v) in PBS 1X (pH 7.4) at 60°C. Once completely dissolved, while still stirring, methacrylic anhydride (276685, Sigma, USA) was slowly added to a final concentration of 8% (v/v). Different levels of methacryloyl and/or methacrylamide functionalization require different concentrations of methacrylic anhydride. After 3 h of reaction, a 5X dilution in 1X PBS was performed and the reacted gelatin was dialyzed against deionized water at 40 °C for 1 week. Daily replacements of fresh deionized water were performed to remove all unreacted methacrylic anhydride during dialysis. Finally, the dialysate mixture was filtered with 20 µm porous filter paper, freeze-dried, and stored at -20 °C for later use.

With this process, the base biomaterial, methacrylate salmon gelatin (sGeIMA), is obtained for the formulation of the dispersant phase solution.

### Example 2. Study of sGelMA viscosity at different concentrations.

In this study, all rheological measurements were taken with an Anton-Paar MCR301 voltage-controlled rheometer (Anton-For, Graz, Austria). For these viscosity measurements, a conical plate with an angle of 0.5° and 25 mm in diameter was used.

Viscosity was measured for salmon gelatin methacrylate (sGeIMA) solutions at different concentrations from 10% to 35% (w/v) through flow sweep with a shear speed between 1 and 1000 s-1 at 25 °C. The results are shown in **Figure 2****.**

The viscosity at 25°C increased as the concentration of the gelatin polymer increased. However, the fluidic behavior was still Newtonian. Table 1 shows the average viscosity of each concentration at 100 s-1.

**Table 1**

| **SGelMA** | **10%** | **20%** | **30%** | **35%** |
|---|---|---|---|---|
| **Viscosity at 100s-1 (cP)** | 9.6±0.3 | 51.5±3.6 | 252±8.5 | 587±48.2 |

### Example 3. Preparation of Gelatin Beads.

Porcine gelatin beads were made by water-in-oil emulsion as described above (Hwang et al., 2010). Briefly, a 10% w/v solution of porcine gelatin (Sigma) was prepared by dissolving 0.5 g in 5 mL of 1X salt phosphate buffer (PBS) at 37 °C with gentle stirring. The pH was adjusted to 7.4 with 1M NaOH. While still hot, the solution was filtered using a 5 µm pore nitrocellulose filter and subjected to a heat-cold sterilization protocol.

The gelatin and mineral oil (Sigma) were preheated to 37°C before the emulsion protocol. In the cell culture hood, 5 mL of the gelatin solution was slowly (1 mL/min) added to 25 mL of mineral oil solution supplemented with 125 µL of Tween 20 (Sigma) under continuous stirring at 600 rpm for 10 minutes. Both the mineral oil and Tween 20 were cell culture quality to ensure the sterility of the beads. The emulsion was then cooled in an ice bath for 10 min at 700 rpm and collected in a 50 mL conical tube containing cold PBS 1X and centrifuged 5 min at 200 x g.

The supernatant was removed and the beads were washed 4 times to remove traces of mineral oil. The beads were kept at 4 °C in PBS 1X supplemented with 100 U/mL penicillin, 100 µg/mL streptomycin until subsequent use.

To prepare the porous hydrogels, the PBS was drained using a 100 µm pore-sized cell filter, and then the beads were included in the respective formulations.

### Example 4. Preparation of the composition of the invention.

To obtain the composition of the invention, 50 mL of a 35% (w/v) solution of sGelMA obtained in example 1 was used and kept in gentle stirring at room temperature. The beads formed in example 3 were poured over this solution, up to the desired concentration (20%, 40% and 60% v/v), forming a stable composition of 2 phases, one dispersant and one dispersed.

Finally, 0.2% (w/v) of lithium phenyl-2,4,6-trimethylbenzoyl phosphine or LAP is added, which is reactive under UV light, so the composition can be handled under normal working light conditions.

### Example 5. Extrusion force study.

Given that the application of the formulation of the invention to form the scaffolds *in situ* must be done through syringes or flexible pipe lines of small diameter, and that allow the formulation to be applied directly on the injury, the extrusion force necessary for the application of the compositions used was studied. For this, the texturometer instrument was used, which was integrated into the plunger of a syringe. With this it was possible to measure the force needed to extrude the compositions through a pipe of a certain length and width.

Study of extrusion strength for sGelMA at different concentrations with and without porcine gelatin beads. The force required to extrude the composition through a channel of:
- 0.58mm inner diameter
- Length 30 cm
- Syringe piston movement speed: 0.2mm/s

Different concentrations between 10 and 35% of functionalized salmon gelatin (sGeIMA) were tested. The summary of the results is in Table 2. The required extrusion force increased as the polymer concentration increased.

**Table 2**

| **SGelMA** | **10%** | **20%** | **30%** | **35%** |
|---|---|---|---|---|
| **N** | 3±0.32 | 8±3.33 | 27±0.63 | 54.51±1.87 |
| **Kg f** | 0.3±0.32 | 0.8±3.33 | 2.7±0.6 | 5.5±1.87 |

Next, the variations in extrusion strength of the sGelMA composition at 35% (w/v) concentration were studied, when different concentrations of porcine gelatin beads are used, at 25°C. Concentrations of 20, 30 and 40% v/v of porcine gelatin beads were evaluated in a dispersing phase of 35% (w/v) of sGeIMA. The results are shown in Table 3.

**Table 3**

| **Gelatin P Beads** | **0%** | **20%** | **40%** | **60%** |
|---|---|---|---|---|
| **N** | 54.5±1.53 | 41.4±6.48 | 16.8±0.87 | 18.7±1.17 |
| **Kg f** | 5.4±1.53 | 4.1±6.48 | 1.7±0.87 | 1.9±1.17 |

The required extrusion force was reduced as the concentrations of porcine gelatin beads increased. Finally, an injectability assessment was performed to determine the force required to inject the precursor formulation in a compression test using a 25G needle (0.5 mm diameter). Therefore, the results showed that the 35% w/v sGelMA solutions possessed injectability values of 55 N. However, a noticeable reduction in strength (20 N vs. 55 N) was shown after adding porcine gelatin beads (40% and 60% v/v) in the concentrated sGelMA hydrogel (**Figure 3**).

### Example 6. Mechanical resistance to compression.

To evaluate the mechanical properties of the scaffold to be formed *in situ,* small discs (6x8 mm) of the compositions of example 4 were polymerized, with a concentration of 10, 20, 30 or 35% (w/v) of sGeIMA. Then, only the concentration of 35% (w/v) of sGelMA was used in the dispersant phase and the dispersed phase (beads) was added at a concentration of 0, 20, 40 or 60% v/v of porcine gelatin beads with respect to the dispersant phase. After photo-crosslinking, the temperature was raised to 37°C so that the porcine gelatin beads dissolved forming the porous scaffold of the invention. The results are shown in **Tables 4 and 5.**

The mechanical compressive strength after photopolymerization for sGelMA at different concentrations with and without porosity was analyzed in a TA.XTplus Texture Analyzer (Stable Micro Systems, UK) with a load cell of 5 kg, at a speed of 2 mm/s, and equipped with a cylindrical probe of 25 cm in diameter. The test determines how much force is needed to break the structure.

**Table 4**

| **SGelMA** | **10%** | **20%** | **30%** | **35%** |
|---|---|---|---|---|
| **KPa** | 11±2.2 | 115±14.2 | 414±59.6 | 714±77.1 |

The level of stiffness increased as the polymer concentration increased.

**Table 5**

| **Gelatin P Beads** | **0%** | **20%** | **40%** | **60%** |
|---|---|---|---|---|
| **KPa** | 695.9±30.5 | 332.1±24.2 | 218.1±21.4 | 100.9±10.8 |

In this study, a gradual reduction in mechanical properties was observed as the concentration of beads increased. These results are also shown in **Figure 4****.** In particular, hydrogels with 60% (v/v) beads drastically reduced their mechanical strength, showing 130 kPa vs. 714 kPa for hydrogels with 35% (w/v) sGelMA with and without beads, respectively. Therefore, a balance between porosity and the desired mechanical properties in the specific application must be considered.

**Figure 5** shows the structural appearance of the porosity of these hydrogels using a scanning electron microscopy image.

As expected, the polymerized gel is stronger at a higher concentration of gelatin in the dispersing phase, and its brittleness increases at a higher concentration of gelatin beads in the dispersed phase. It is notable that hydrogels with the highest concentration of sGelMA reached 714 kPa, which is very close to the compression modulus of human cartilage.

### Example 7. Cytocompatibility of porous and non-porous sGelMA scaffolds.

The potential cytotoxicity of UV crosslinking byproducts and gelatin beads was evaluated using an indirect assay employing 3T3 fibroblasts and hydrogel extracts. The extracts were obtained after subjecting 1 volume of culture medium to 1 volume of hydrogel for 24 h at 37°C. The supernatant was considered as an extract and was used as a culture medium in cytotoxicity assays.

Porous hydrogel extracts did not affect metabolic activity (**Figure 6****.A**) or cell viability (**Figure 6****.B**) compared to 35% of sGelMA without pores, meaning that the beads allow for an effective injectability/porosity system without inducing any toxicity.

Since the delivery of viable cells encapsulated within formed hydrogels is a desired capability for tissue engineering applications, the feasibility of cell encapsulation using sGelMA porous hydrogels was examined. Encapsulated 3T3 fibroblasts were cultured for 6, 24, and 48 hours and cell viability was assessed using the *LIVE*/*DEAD* assay. Hydrogel porosity improved cellular metabolic activity at 24 to 48 hours after encapsulation in hydrogels of 35% (w/v) sGelMA (**Figure 7****.A**). In this context, a remarkable recovery of viability was shown in all porous hydrogels with viabilities greater than 70% after 24 hours of culture (**Figure 7****.B**).

### Example 8. In vivo injection of the sGelMA porous scaffold.

An *in vivo* proof of concept of an injectable hydrogel was carried out at the facilities of the Cells for Cells vivarium, in accordance with the guidelines of the Bioethics Committee of the Institutional Committee for the Care and Use of Animals of the Universidad de los Andes, Santiago, Chile *(Los Andes University, Santiago, Chile).*

C57BL/6 mice six to eight weeks of age (The Jackson Laboratory) were anesthetized with vaporized sevoflurane (Baxter). Two hydrogels per formulation (35% sGelMA hydrogel solution and 35% sGelMA hydrogel solution containing 40% (w/v) beads) were injected subcutaneously (400 µL) along each flank of the mice. The photo-crosslinking was performed in the solution by direct injection of a customized device that allowed the insertion of the needle containing the UV optical fiber.

Prior to subcutaneous hydrogel implantation, an extrusion strength test was performed with an 18 G x 1" (1.2 mm diameter) needle cannulated to a gauge tube (inside diameter: 1 mm) to emulate an arthroscopic procedure. Our results showed that less than 5 N were required to inject sGelMA solutions (**Figure 8****.B**), being appropriate for *in situ* surgical procedures.

The in-situ injection process required two steps, including injection with the SGelMA solution and subsequent insertion of the needle with ultraviolet light to cross-link the solution (Figure 8.A and **Figures 8****. C-D**)**.** No evidence of sepsis, infection, or pain was identified in the animals during the period between injection and collection of the hydrogel.

The mice were kept on food and water *ad libitium* under a 12/12 h light/dark cycle at 22±1°C. The resulting hydrogels were collected after days 1 and 14 post-injection, respectively (**Figure 8****.E** and **Figure 8****.F**) for further tissue processing, histological studies, and structural analyses. The mice were sacrificed by intraperitoneal overdose of xylazine and ketamine.

Hydrogel samples were fixed with 10% neutral phosphate-buffered formalin (Sigma Aldrich), dehydrated with an increasing concentration of alcohols and xylene, and included in paraffin in an automatic tissue processor (STP 120 Spin Tissue Processor, Thermo Scientific^{™}). Samples were cut into 5 µm sections, stained with hematoxylin-eosin (HE), and observed under the light microscope to characterize porosity formation and integrity.

The porous structure formed *in situ* was visualized by electron microscopy (**Figure 8****.G** and **8.K**) and histology (**Figure 8****.I** and **Figure 8****.M**) compared to nonporous hydrogels (**Figure 8****.H,** **Figure 8****.J,** **Figure 8****.L, and** **Figure 8****.N**).

Surprisingly, 14 days after injection, porous sGelMA showed infiltration and cell colonization indicated by the presence of suspected mouse fibroblasts on electron microscope **(****Figure 8.K****)** and histology (**Figure 8****.M**) images. In contrast, nonporous sGelMA revealed no cellular infiltration (**Figure 8****.L,** **Figure 8****.N**).

### Example 9. Use of the injectable porous sGelMA scaffold in 3D bioprinting

3D printability of salmon GelMA bioink with porcine gelatin beads.

After testing the feasibility of forming porous sGelMA hydrogels during injection and on-site illumination, the use of salmon GelMA with porcine gelatin beads as bioink for 3D printing was also evaluated. Therefore, we printed a 3D structure using an extrusion-based 3D printer, Fab@Home model 3 (**Figure 9****.A**). During the deposition of the printing material, the sGelMA 35% bioink with beads (40% v/v) flowed easily without obstructions, demonstrating the feasibility of using this bioink under mild conditions. The 3D construction showed good doughnut-shaped fidelity (**Figure 9****.B**) and the stability of the beads was maintained after printing, as evidenced by confocal microscopy (**Figure 9****.C and** **Figure 9****.D**).

### Example 10. Injectable porous scaffolds that carry live chondrocytes.

Chondrocytes derived from patients with osteoarthritis (OAC) were isolated from the femoral head of patients undergoing hip replacement. The samples were processed and a cell suspension of OAC at a density of 30,000 cells/cm2 was seeded in a cell culture dish. The medium was replaced 2-3 times per week during cell culture. When OACs reached 80-90% confluence, they were separated from the plate using 0.5% trypsin/EDTA and isolated by centrifugation for subsequent resuspension and experiments. Cells were only used until subculture 2 through 3 (passage 2-3). All cell cultures were kept in an incubator humidified at 37°C and 5% CO2.

After cell isolation and centrifugation, the cells were suspended in the respective formulations, including beads, sGelMA, PBS 1X, and 0.2% (w/v) LAP (Sigma) photoinitiator. Where sGelMA was at a concentration of 20%, 25% or 35% (w/v), all with 40% (v/v) gelatin beads.

A volume of 100 µL of a 7.5 x 10⁶ cell/mL suspension was poured into cylindrical PDMS molds (6 mm ϕ, 5 mm h) and then exposed to 365 nm filtered UV light with an intensity of 261 mW/cm2 (OmniCure^{®} S2000, Excelitas Technologies, USA) at a distance of 20 cm and for 20 sec per side.

Cellularized hydrogels were deposited in a 12-well plate (1 hydrogel per well) and 2 mL of chondrogenic differentiation medium (DMEM supplemented with 0.2 mM ascorbic acid 2-phosphate, 0.5% (v/V) bovine serum albumin, 1% v/v insulin-transferrin selenium mixture (ITS-X, Corning), 4 mM L-glutamine, 100 U/ml penicillin, 100 µg/mL streptomycin, 1 mM sodium pyruvate, and 5 ng/mL TGF-B2) were added and changed twice weekly.

The day after encapsulation, the LIVE/DEAD^{®} stain showed a similar morphology and viability between the 3 formulations, where the predominant morphology was rounded, which was to be expected because they did not have enough time to remodel the matrix and adapt to this 3D environment (**Figure 10****.B**). After 35 days, the morphology is different. In G20B (20% (w/v) sGelMA, 40% (v/v) beads) the cells spread and acquired a fibroblastoid phenotype, while in G35B (35% (w/v) sGelMA, 40% (v/v) beads), the cells remained mostly rounded (**Figure 10****.B**). Cells in G25B (25% (w/v) sGelMA, 40% (v/v) beads) were between the two phenotypes. We quantified live and dead cells on day 1 and 35 and detected no differences on day one, but with high intra-variation because some regions in the hydrogels might have received more stress than others, which was reflected in all 3 conditions. After 35 days, there was a significant decrease in G35B viability, with more cell death (**Figure 10****.C**). The number of cells at the end of the trial was similar between formulations (data not shown). Another measure of feasibility was the determination of the metabolic activity of the OACs throughout the 5 weeks of the trial, at the beginning (day 1) G35B started lower than G20B and G25B but there was an increase in metabolic activity under all conditions at 14 days. reaching a plateau on day 35, where G35B was significantly lower than the other conditions, either because of a lower number of cells or because of a decreased metabolism (**Figure 10****.D**).

There were considerable differences between the cell morphology of the 3 formulations. For the measurement of the fibroblastoid phenotype (cell propagation), hydrogel slides were stained for smooth muscle alpha-actin (αSMA) and cell circularity was determined on day 1 and day 35. According to the LIVE/DEAD^{®} images, the OAC G20Bs changed their morphology, showing multiple cell protrusions like a fibroblast, and with a decrease in cell circularity at the end of the experiment. A similar result was observed for G25B, but for G35B the circularity of the cells did not decrease as much as in the case of the other formulations, suggesting a morphology similar to that of chondrocytes or an inability to adapt and remodel this formulation (**Figure 10****.E-F**).

The distribution of OAC was homogeneous throughout the hydrogel, but there were considerable differences in cell morphology under all 3 conditions, as determined by H&E staining. In G20B, OACs had a mainly fibroblastoid appearance, forming large clusters of deep pink cells (eosinophils) and surrounded by a pink fibrous halo, suggesting a deposit of ECM or ectracellular matrix. A similar distribution was observed in G25B, but with less fibrous structures, and OACs were also detected within lagoon-like structures reminiscent of cartilage tissue. In G35B, fibrous deposits were scarce and the cells were contained in the same gap-like structures just described (**Figure 10****.G**).

These results prove that the method of invention and the scaffold formed allow for the incorporation of living cells, and the cells survive the cross-linking process and are also functional.

## Claims

1. Method for generating a porous injectable scaffold as a biological matrix **CHARACTERIZED in that** it includes the stages of:
i) providing a liquid composition of 2 phases at a temperature below 25°C, of Newtonian behavior, where the composition comprises:
a first liquid dispersant phase at room temperature formed by a gelatin with a low melting point, less than 15°C, determined by the fact that it has a proline and hydroxyproline content of 18% or less with respect to the total amino acid content; which is additionally functionalized with methacryloyl or methacrylamide groups; and a photoinitiator;
and a second dispersed phase, of solid-state microdroplets or beads of a gelatin solution with a melting point greater than 25°C, determined by having a proline and hydroxyproline content of 20% or more of the total amino acid content;
(ii) extruding the solution provided at the site where the porous scaffold will be formed;
(iii) initiating the polymerization of the liquid dispersant phase by applying light radiation;
(iv) raising the temperature to 35-40°C and allow melting of the beads of the dispersed phase; and
v) obtaining a porous scaffold by jellification of the dispersing phase and discarding by melting and/or diffusion of the dispersed phase.

2. Method according to claim 1 **CHARACTERIZED in that** the liquid dispersant phase additionally comprises active compounds such as drugs, growth and/or migration factors, cells, organelles, antibodies, peptides, vesicles, cell derivatives and/or oligonucleotides.

3. Method according to claim 1 **CHARACTERIZED in that** the low-melting point gelatin of the dispersing phase is at a concentration between 10 and 50 % w/v.

4. Method in accordance with claim 3 **CHARACTERIZED in that** the degree of functionalization with methacryloyl or methacrylamide groups of the gelatin with a low melting point or dispersing phase is 30% to 100% of lysine residues in the amino acid chain of said gelatin.

5. Method according to claim 1 **CHARACTERIZED in that** the gelatin beads of the dispersed phase have a concentration of between 5% and 30% w/v of gelatin; and they are in a concentration of between 10 and 80% v/v with respect to the dispersing phase.

6. Method according to claim 1 **CHARACTERIZED in that** gelatin beads have a diameter of between 50 and 500 µm.

7. Method according to claim 1 **CHARACTERIZED in that** in step ii) the composition is injected directly into the site where the formation of the recipient scaffold, tissue, or lesion is required, and in stage iii) it is polymerized *in situ.*

8. Method according to claim 7 **CHARACTERIZED in that** it is injected into a lesion, and given the Newtonian character of the composition, it is easily injected, adapts and completely covers the lesion, and when polymerized, a porous scaffold perfectly adapted to the required shape is formed.

9. Method according to claim 7 **CHARACTERIZED in that** the natural temperature of the recipient tissue allows the fusion of the beads of the dispersed phase.

10. Method in accordance with claim 1 **CHARACTERIZED in that** in stage ii) the composition is extruded by means of a 3D printing system, which incorporates a light emission application system for the polymerization of stage iii.

11. Method according to claim 10 **CHARACTERIZED in that** the application of heat to raise the temperature, according to step iv) can be done *in vitro,* with a heat source.

12. Method according to claim 10 **CHARACTERIZED in that** the application of heat to raise the temperature, stage iv), can be performed naturally by the temperature of the recipient, by arranging the polymerized scaffold in a 3D printing system on a lesion or recipient tissue.

13. Method according to claim 1 **CHARACTERIZED in that** low-melting gelatin is derived from organisms of the genus Salmo or Oncorhynchus.

14. Method according to claim 1 **CHARACTERIZED in that** the solid gelatine at room temperature is pig or bovine gelatin.

15. Porous scaffold formed according to the method described in any of claims 1 to 14, **CHARACTERIZED in that** it is formed of polymerized gelatin with pores between 50 and 500 µm in diameter.

16. Porous scaffold of claim 15 **CHARACTERIZED in that** it additionally comprises active compounds such as drugs, growth and/or migration factors, cells, organelles, antibodies, peptides, vesicles, cell derivatives and/or oligonucleotides.

17. Use of claim 15 scaffold **CHARACTERIZED in that** it serves as a biological support for tissue regeneration/generation.

18. Use of claim 16 **CHARACTERIZED in that** the tissue regeneration is in a joint injury, cartilage injury, bone injury, ulcerative injury, ligament injury, organ injury, spinal cord injury, or soft tissue injury.

19. Use of claim 16 **CHARACTERIZED in that** the tissue regeneration being a reconstitution of skin tissue, muscle tissue, soft tissue, or post-surgical replacement tissue.

20. Use of claim 15 scaffold **CHARACTERIZED in that** it serves as a biological matrix as a support for cells, for cellular invasion.

21. Use of claim 15 scaffold **CHARACTERIZED in that** it serves as an acellular biological matrix, biological matrix as a mechanical support, biological matrix for active components.
